# EUROPÄISCHE PATENTSCHRIFT

(11) **EP 2 686 289 B1**
(45) Veröffentlichungstag und Bekanntmachung des Hinweises auf die Patenterteilung: **08.05.2019**
(21) Anmeldenummer: 12708534.8
(22) Anmeldetag: 07.03.2012
(51) Int. Cl.: B01J 21/12, C07C 1/213, C07C 11/09, B01J 37/10, B01J 37/28, B01J 23/00, B01J 23/04, B01J 27/16, B01J 27/18, B01J 35/00, B01J 35/10

(54) **MISCHOXIDZUSAMMENSETZUNGEN UND VERFAHREN ZUR HERSTELLUNG VON ISOOLEFINEN**
MIXED OXIDE COMPOSITI0NS AND METHODS FOR PRODUCTION OF ISOOLEFINS
COMPOSITION D'OXYDES MIXTES ET PROCÉDÉ DE PRÉPARATION D'ISO-OLÉFINES

(30) Priorität: 16.03.2011 DE 102011005608
(43) Veröffentlichungstag der Anmeldung: 22.01.2014
(73) Patentinhaber: Evonik Degussa GmbH, 45128 Essen (DE)
(72) Erfinder: WINTERBERG, Markus, 45711 Datteln (DE); BÖING, Christian, 50679 Köln (DE); MASCHMEYER, Dietrich, 45657 Recklinghausen (DE); NAU, Asli, 58300 Wetter (Ruhr) (DE); ZANTHOFF, Horst-Werner, 45481 Mülheim a.d. Ruhr (DE); QUANDT, Thomas, 45772 Marl (DE); SCHULZE ISFORT, Christian, 63694 Limeshain (DE)
(74) Vertreter: Evonik Patent Association
(86) Internationale Anmeldenummer: PCT/EP2012/053835
(87) Internationale Veröffentlichungsnummer: WO 2012/123292

(56) Entgegenhaltungen:
- EP-A1- 1 266 864
- EP-A2- 1 186 628
- US-A- 5 227 564

## Beschreibung

Die vorliegende Erfindung betrifft Mischoxidzusammensetzungen, deren Verwendung als Katalysator zur Spaltung von Alkyl-tert.-alkylethern oder tertiären Alkoholen sowie ein Verfahren zur Spaltung von Alkyl-tert.-alkylethern oder tertiären Alkoholen in Isoolefine und Alkohol oder Wasser.

Isoolefine, wie z. B. Isobuten, sind wichtige Zwischenprodukte für die Herstellung einer Vielzahl organischer Verbindungen. Isobuten ist beispielsweise Ausgangsstoff für die Herstellung von Butylkautschuk, Polyisobutylen, Isobuten-Oligomeren, verzweigten C₅-Aldehyden, C₅-Carbonsäuren, C₅-Alkoholen und C₅-Olefinen. Weiterhin wird es als Alkylierungsmittel, insbesondere zur Synthese von tert.-Butylaromaten, und als Zwischenprodukt für die Erzeugung von Peroxiden eingesetzt. Darüber hinaus kann Isobuten als Vorstufe für Methacrylsäure und deren Estern verwendet werden.

In technischen Strömen liegen Isoolefine meist zusammen mit anderen Olefinen und gesättigten Kohlenwasserstoffen mit gleicher Kohlenstoffatomzahl vor. Aus diesen Gemischen sind die Isoolefine allein mit physikalischen Trennmethoden wirtschaftlich nicht abtrennbar.

Beispielsweise liegt Isobuten in üblichen technischen Strömen zusammen mit gesättigten und ungesättigten C₄-Kohlenwasserstoffen vor. Aus diesen Gemischen kann Isobuten wegen der geringen Siedepunktsdifferenz bzw. des geringen Trennfaktors zwischen Isobuten und 1-Buten durch Destillation nicht wirtschaftlich abgetrennt werden. Daher wird Isobuten aus technischen Kohlenwasserstoffen häufig dadurch gewonnen, dass Isobuten zu einem Derivat umgesetzt wird, das sich leicht vom übrigen Kohlenwasserstoffgemisch abtrennen lässt, und dass das isolierte Derivat zu Isobuten und Derivatisierungsmittel zurückgespaltet wird.

Üblicherweise wird Isobuten aus C4-Schnitten, beispielsweise der C4-Fraktion eines Steamcrackers, wie folgt abgetrennt. Nach Entfernung des größten Teils der mehrfach ungesättigten Kohlenwasserstoffe, hauptsächlich des Butadiens, durch Extraktion(-sdestillation) oder Selektivhydrierung zu linearen Butenen wird das verbleibende Gemisch (Raffinat I oder selektiv hydriertes Crack-C₄) mit Alkohol oder Wasser umgesetzt. Bei der Verwendung von Methanol entsteht aus Isobuten Methyl-tert.-butylether (MTBE), bei der Verwendung von Ethanol Ethyl-tert.-butylether (ETBE) und bei Einsatz von Wasser tert.-Butanol (TBA). Nach ihrer Abtrennung können diese Derivate in Umkehrung ihrer Bildung zu Isobuten gespalten werden.

Die Spaltung von Alkyl-tert.-butylethern (ATBE) zu den entsprechenden Isoolefinen und Alkoholen sowie die Spaltung von tertiären Alkoholen zu den entsprechenden Isoolefinen und Wasser kann in Gegenwart saurer oder basischer Katalysatoren in der Flüssigphase bzw. Gas/Flüssig-Mischphase oder in der reinen Gasphase durchgeführt werden.

Es sind in der Literatur eine Vielzahl von Katalysatoren für die Gasphasenspaltung von Alkyl-tert.-alkylethern (ATAE) und tertiären Alkoholen zu den entsprechenden Isoolefinen sowie Alkohol oder Wasser beschrieben. Dies gilt insbesondere für Katalysatoren, die zur Spaltung von Methyl-tert.-butylether (MTBE) genutzt werden.

Als Katalysatoren werden dabei meistens Metalloxide mit einer Summenformel MₐOₓ, Metallmischoxidzusammensetzungen mit Summenformeln MₐM_{b}MₙO_{y}, wobei die Indices a, b, n, x, y ganzzahlig oder rationale Zahlen sein können, insbesondere solche, die M = Si oder M =Al enthalten, Säuren auf Metalloxidträgern oder Metallsalze verwendet.

In US 4,254,290 werden als Spaltkatalysatoren beispielsweise SiO₂/Al₂O₃ oder WO₃/Al₂O₃ beschrieben. In US 4,320,232 und US 4,521,638 werden zur Spaltung von tertiären Ethern Katalysatoren, bestehend aus Phosphorsäure auf Trägern, beansprucht. Aluminiumoxid auf Kieselgel wird in US 4,398,051 als Spaltkatalysator genutzt. Für den gleichen Zweck werden in den beiden Patentschriften US 4,357,147 und US 5,254,785 Zeolithe verwendet.

In JP 59010528 wird als Spaltkatalysator sulfatiertes Titandioxid oder Zirkondioxid verwendet. In US 5,607,992 wird zur Spaltung von Ethern ein Zirkoniumoxid/Ceroxid-Katalysator, in US 6,124,232 Zirkoniumoxid/Wolframoxid, in US 6,162,757 ein Mischoxid von Zirkonium und Seltenen Erden eingesetzt.

In WO 2005-066101 wird ein Katalysator mit allgemeinen Summenformel XₘYₙZₚO_{q} beansprucht, wobei X mindestens ein Element der vierten Gruppe des Periodensystems der Elemente, Y mindestens ein Metall aus der dritten und/oder sechsten Gruppe und Z mindestens ein Element aus der siebten, achten oder elften Gruppe ist.

In JP 1993-229965 wird ein Katalysator mit der Summenformel SiₐX_{b}Y_{c}Z_{d}Oₑ beansprucht. (Hier stehen Si und O jeweils für Silizium und Sauerstoff; X ist zumindest ein Element, das aus der Gruppe, bestehend aus Titan und Zirkonium, ausgewählt ist; Y ist ein Element, das aus der Gruppe, bestehend aus Magnesium und Calcium ausgewählt ist; Z ist zumindest ein Element, das aus der Gruppe, bestehend aus Natrium, Kalium, Chlor und Schwefel ausgewählt ist; a, b, c, d und e zeigen das Atomverhältnis der einzelnen Elemente an. Wenn a = 1 ist, dann sind b =0.001 bis 10, c =0.0001 bis 5, d =0 bis 1; e ist die notwendige Sauerstoffatomzahl, um den Atomwert der vorstehend genannten einzelnen Bestandteile zu erfüllen).

In US 5,227,564 wird ein Gasphasenprozess zur Herstellung von Isoolefin durch Spaltung der entsprechenden Alkyl-tert.-alkylethern mit Hilfe eines Katalysators, der aus einer Mischung eines zeolithischen Materials, das ein Silizium-zu-Aluminium-Verhältnis von größer 5 aufweist, mit einem röntgenamorphen Oxid der Elemente Silizium, Aluminium oder einem Mischoxid beider Elemente besteht, beschrieben.

In US 5,171,920 wird unter anderem ein Mischoxid der Elemente Silizium, Aluminium und Magnesium als Katalysator für die Etherspaltung beschrieben. Die Herstellung geschieht derart, dass zunächst Siliziumdioxid mit einer wässrigen Magnesiumnitrat-Lösung getränkt/imprägniert wird, nach einer Zwischentrocknung erfolgt eine weitere Tränkung/Imprägnierung mit einer wässrigen Aluminiumnitrat-Lösung. Anschließend wird nach Vortrocknung kalziniert. Der Aluminiumoxid-Gehalt beträgt 0.37 Massen-% (gerechnet als Al₂O₃) und der Magnesiumoxid-Gehalt beträgt 7.7 Massen-% (gerechnet als MgO).

In EP 0 045 159 wird unter anderem ein Mischoxid der Elemente Silizium und Aluminium mit einem Aluminiumoxid-Gehalt von 13 Massen-% (gerechnet als Al₂O₃) als Katalysator für die Spaltung von Alkyl-tert.-alkylethern beschrieben. Die Herstellung geschieht derart, dass ein kommerzielles gefälltes Silizium-Aluminium-Mischoxid mit dem entsprechenden Verhältnis der Elemente gemahlen und anschließend kalziniert wird.

In der EP 1 266 864 A1 wird ein mit Aluminiumoxid dotiertes, pyrogen hergestelltes Siliziumdioxid offenbart, welches mittels Sprühgranulation hergestellt wird. Die so hergestellten mit Aluminiumoxid dotierten Siliziumdioxide können als Katalysatoren oder Katalysatorträger für verschiedene Reaktionen, beispielsweise Hydrierungen, Dehydrierungen, Hydratisierungen, Dehydratisierungen oder Isomerisierungen eingesetzt werden.

Die EP 1 186 628 A2 offenbart Rohstoffdispersion für die Herstellung von Polyestern, wobei die Dispersionen ein Silica-Pulver in einem Glykol umfassen. Die Rohstoffdispersionen können zur Bildung von Polyesterschichten, die Silica-Teilchen enthalten, verwendet werden.

In der US 5,227,564 A wird ein Verfahren zur Herstellung tertiärer Olefine aus entsprechenden tertiären Alkylethern offenbart. Als Katalysator wird ein Zusammensetzung verwendet, die Alumosilikat-Zeolith und ein Bindemittel umfasst.

DE 292 486 9 beschreibt einen Katalysator für die Spaltung von Alkyl-tert.-alkylethern auf Basis von kristalliner Kieselsäure, welche mit ggf. mit Metalloxiden modifiziert wird. Unter anderem wird eine Modifizierung mit 0.2 Massen-% Aluminiumoxid (gerechnet als Al₂O₃) beschrieben. Die Herstellung geschieht derart, dass Tetraethyl-orthosilikat in Gegenwart von Aluminiumnitrat-nonahydrat gefällt, auskristallisiert und kalziniert wird.

In EP 0 589 557 wird u. a. ein Mischoxid der Elemente Silizium, Aluminium und Magnesium als Katalysator für die Etherspaltung beschrieben. Bei seiner Herstellung wird in einem ersten Schritt ein kommerziell erhältliches durch Fällung hergestelltes Silizium-Aluminium-Mischoxid mit einer wässrigen Magnesiumsalzlösung in der Weise imprägniert, das während der Imprägnierung der pH-Wert der Tränklösung durch Zugabe einer Base auf einen pH-Wert von 7 bis 11 eingestellt werden kann. Um besonders aktive und selektive Katalysatoren zu erhalten, sind zum Teil Imprägnierzeiten von über 200 h erforderlich.

EP 1 894 621 A1 beschreibt ein Gasphasenverfahren zur Herstellung von Isoolefinen mit einem Alkali- und/oder Erdalkalimetall-dotierten Silizium-Aluminium-Mischoxid als Katalysator, der durch behandeln des kommerziell erhältlichen durch Fällung hergestellten Silizium-Aluminium-Mischoxids mit einer wässrigen Alkali- oder Erdalkalimetallsalzlösung unter sauren Bedingungen und anschließender Kalzinierung hergestellt wird. Mit dem beschriebenen Katalysator werden bei einem Umsatz von ca. 85% hohe Isobuten-Selektivitäten von > 99% und ebenfalls hohe Methanol-Selektivitäten von > 99% erzielt. Mit steigender Versuchsdauer sinkt der Umsatz jedoch bei sonst gleich bleibenden Versuchsbedingungen (Temperatur, Druck, VWZ, Katalysatormenge, Feed-Zusammensetzung) ab. Um weiterhin einen hohen Umsatz gewährleisten zu können, muss man kontinuierlich die Temperatur anheben. Dadurch steigt jedoch die Masse der Nebenkomponenten und die Selektivität zu den Hauptkomponenten sinkt. Insbesondere die Bildung von Dimethylether steigt dabei an.

Die Spaltung in der Flüssigphase bzw. Gas/Flüssigphase hat den Nachteil, dass die entstehenden Produkte, gelöst in der Flüssigphase, leichter Nebenreaktionen eingehen können. Beispielsweise kann das bei der Spaltung von MTBE entstehende Isobuten durch sauer katalysierte Dimerisierung oder Oligomerisierung unerwünschte C₈- und C₁₂-Komponenten bilden. Bei den unerwünschten C₈-Komponenten handelt es sich hauptsächlich um 2,4,4-Trimethyl-1-penten und 2,4,4-Trimethyl-2-penten. Darüber hinaus setzt sich besonders an basischen Katalysatoren, ein Teil des bei der Spaltung entstehenden Methanols unter Wasserabspaltung zu Dimethylether um. Wird die Reaktion nicht unter Drücken oberhalb des Sättigungsdampfdruckes des Reaktionsgemisches durchgeführt, um diesen Problemen entgegenzuwirken, so ist ein zusätzliches Lösemittel notwendig.

In der Gasphase kann die Bildung von Nebenreaktionen durch Reaktion der Spaltprodukte mit sich selbst aufgrund deren geringerer Konzentrationen im Vergleich zur Spaltung in der Flüssigphase zurückgedrängt werden. Es können allerdings wegen der höheren Spalttemperaturen andere Nebenreaktionen auftreten. Bei der Gasphasenspaltung sind daher Katalysatoren erforderlich, die mit sehr hoher Selektivität die Spaltung von tertiären Alkylethern oder tertiären Alkoholen in Isoolefin sowie Alkohol oder Wasser katalysieren, jedoch keine Nebenreaktionen, wie beispielsweise C-C-Spaltung oder Dehydrierung sowie C-C-Kupplungsreaktionen oder Etherbildung der entstehenden Alkohole, begünstigen. Weiterhin sollen diese Katalysatoren hohe Raum-Zeit-Ausbeuten ermöglichen und eine hohe Standzeit aufweisen. Darüber hinaus ist eine Spaltung des Edukts mit möglichst hoher Selektivität für das entstehende Isoolefin bei einem Druck größer 0,3 MPa(abs) wünschenswert.

Die bekannten Katalysatoren weisen bei der Spaltung von Alkyl-tert.-alkylethern oder tertiären Alkoholen in Isoolefin sowie Alkohol bzw. Wasser einen oder mehrere Nachteile auf:
a) Zu starke Bildung von unerwünschten Nebenprodukten wie z.B. Dimethylether oder Oligomere der Produktolefine.
b) Geringe Standzeit des Katalysators.
c) Verstärkte Bildung von Nebenprodukten bei Anhebung der Reaktionstemperatur zum Ausgleich von Aktivitätsverlust.
d) Aufwändige und damit kostenintensive Herstellung des Katalysators.
Es bestand daher die Aufgabe, einen Spaltkatalysator bereitzustellen, der einen oder mehrere dieser Nachteile nicht aufweist.

Überraschenderweise wurde nun gefunden, dass bestimmte Silizium- und Aluminiumaufweisende Mischoxidpulver, bei deren Herstellung ein flammenhydrolytisch oder pyrogen hergestelltes Silizium-Aluminium-Mischoxid verwendet wurde, eine hohe katalytische Aktivität für die Spaltung von Alkyl-tert.-alkylethern oder tertiären Alkoholen in Isoolefin sowie Alkohol bzw. Wasser bei gleichzeitig sehr geringer Bildung von unerwünschten Nebenprodukten aufweisen. Ggf. kann es sinnvoll sein, diese Mischoxide mit Alkalimetall- oder Erdalkalimetalloxiden zu dotieren.

Die Mischoxide werden nach Art der Flammenhydrolyse hergestellt, beschrieben unter anderem in DE 198 47 161 A1, DE 196 50 500 A1, EP-A 0850 876 und Koth et al., Chem.-Ing.-Tech. 1980, 52, 628ff.

Dabei werden beim sog. "co-fumed-Verfahren" flüchtige Silizium- und Aluminium-Verbindungen, meist Siliziumtetrachlorid und Aluminiumtrichlorid, in eine Knallgasflamme aus Wasserstoff und Sauerstoff bzw. Luft eingedüst. Die flüchtigen Silizium- und Aluminium-Verbindungen werden durch das in der Knallgasflamme entstandene Wasser hydrolysiert und es bildet sich das Mischoxid sowie die Säure des Gegenions der Silizium- und Aluminium-Verbindungen.

Beim alternativ angewendeten Dotierverfahren wird in eine Knallgasflamme, in der ein Oxid, z.B. Siliziumoxid, aus einer flüchtigen Verbindung, z.B. Siliziumtetrachlorid, durch Flammenhydrolyse erzeugt wird, ein Aerosol eingespeist, wobei sich in diesem Aerosol ein Salz des zu dotierenden Elementes, z.B. Aluminium, befindet und so das entsprechende Mischoxid bildet.

Die entstandenen Koppelprodukte werden anschließend bei beiden Verfahren in verschiedenen Schritten, wie unter anderem in DE 198 47 161 A1, DE 196 50 500 A1, EP-A 0850 876 und Koth et al., Chem.-Ing.-Tech. 1980, 52, 628ff beschrieben, entfernt. Die flammenhydrolytisch hergestellten Oxide bzw. Mischoxide zeichnen sich durch folgende Besonderheiten aus:
1. Hohe chemische Reinheit,
2. definierte, kugelförmige Primärteilchen,
3. praktisch keine innere Oberfläche.

Des weiteren zeichnen sich die erfindungsgemäßen Silicium-Aluminium-Mischoxidpulver dadurch aus, dass sie überwiegend oder vollständig in Form von aggregierten Primärpartikeln vorliegen und bei denen
a. das Gewichtsverhältnis von (Al₂O₃/SiO₂)ₜₜₗ im Gesamtprimärpartikel 0,002 bis 0,05, bevorzugt 0,003 bis 0,015, besonders bevorzugt 0,005 bis 0,01,
b. das Gewichtsverhältnis (Al₂O₃/SiO₂)_{Oberfläche} der Primärpartikel in einer oberflächennahen Schicht mit einer Dicke von 5 nm kleiner als im Gesamtprimärpartikel und
c. die BET-Oberfläche 50 bis 250 m²/g, bevorzugt 100 bis 200 m²/g, ist.

Die erfindungsgemäße Silicium-Aluminium-Mischoxidpulver zeichnet sich unter anderem dadurch aus, dass der Anteil des Aluminiumoxides gegenüber dem Siliciumdioxid sehr gering ist und das Gewichtsverhältnis (Al₂O₃/SiO₂)Oberfläche der Primärpartikel in einer oberflächennahen Schicht kleiner als im Gesamtprimärpartikel ist. Dies bedeutet, dass die Aluminiumoxidkonzentration an der Oberfläche weiter reduziert ist. Der Gesamtprimärpartikel schließt den Anteil an Siliciumdioxid und Aluminiumoxid in der oberflächennahen Schicht mit ein. Bevorzugt kann ein erfindungsgemäßes Silicium-Aluminium-Mischoxidpulver sein, bei dem (Al₂O₃/SiO₂)ₜₜₗ / (Al₂O₃/SiO₂)Oberfläche 1,3 bis 20, bevorzugt 1,4 bis 10 und besonders bevorzugt 1,6 bis 5 ist, wobei "ttl." für den Gesamtprimärpartikel steht.

In einer bevorzugten Ausführungsform der Erfindung weist das Silicium-Aluminium-Mischoxidpulver ein Gewichtsverhältnis von (Al₂O₃/SiO₂)ₜₜₗ von 0,005 bis 0,015, ein Verhältnis (Al₂O₃/SiO₂)ₜₜₗ / (Al₂O₃/SiO₂)Oberfläche von 1,3 bis 20 und eine BET-Oberfläche von 100 bis 200 m²/g auf.

Unter Mischoxidpulver soll eine innige Mischung der Mischoxidkomponenten Aluminiumoxid und Siliciumdioxid auf atomarer Ebene verstanden werden, bei der die Primärpartikel auch Si-O-Al-Bindungen aufweisen. Die Oberflächen dieser Primärpartikel sind weitestgehend oder vollständig frei von Poren.

Bevorzugt können solche erfindungsgemäßen Silicium-Aluminium-Mischoxidpulver sein, die durch Flammenhydrolyse und/oder Flammenoxidation von Silicium- und Aluminiumverbindungen in einer Flamme, erzeugt durch die Reaktion von Wasserstoff und Sauerstoff, erhalten werden. Diese Pulver werden als "pyrogen" oder "fumed" beschrieben. Bei der Reaktion werden zunächst hochdisperse Primärpartikel gebildet, die im weiteren Reaktionsverlauf zu Aggregaten zusammenwachsen und diese weiter zu Agglomeraten zusammenlagern können.

Das Gewichtsverhältnis auf der Oberfläche kann zum Beispiel durch röntgeninduzierte Photoelektronen-Spektroskopie (XPS-Analyse) des Pulvers bestimmt werden. Zusätzliche Information über die Oberflächenzusammensetzung kann durch energiedispersive Röntgenstrahlung (TEM-EDX-Analyse) von einzelnen Primärpartikeln bestimmt werden.

Das Gewichtsverhältnis im Gesamtprimärpartikel wird durch chemische oder physikalisch-chemische Methoden, z.B. Röntgenfluoreszenzanalyse, des Pulvers bestimmt.

Weiterhin wurde gefunden, dass es vorteilhaft sein kann, wenn das Silicium-Aluminium-Mischoxidpulver eine Dibutylphthalat-Zahl, in g Dibutylphthalat (DBP)/100 g Mischoxid, von 300 bis 350 aufweist. Die DBP-Zahl stellt ein Maß für die Struktur von Aggregaten dar. Niedrige Zahlen korrespondieren mit einer niedrigen Struktur, hohe Zahlen mit einer hohen Struktur. Der bevorzugt Bereich von 300 bis 350 entspricht einer hohen Struktur. Bei der DBP-Absorption wird die Kraftaufnahme, beziehungsweise das Drehmoment (in Nm), der rotierenden Schaufeln des DBP-Meßgerätes bei Zugabe definierter Mengen von DBP, vergleichbar einer Titration, gemessen. Dabei ergibt sich für das erfindungsgemäße Pulver ein scharf ausgeprägtes Maximum mit einem anschließenden Abfall bei einer bestimmten Zugabe von DBP. Die Dibutylphthalatabsorption kann beispielsweise mit einem Gerät RHEOCORD 90 der Fa. Haake, Karlsruhe gemessen werden. Hierzu werden 12 g des Silicium-Aluminium-Mischoxidpulvers auf 0,001 g genau in eine Knetkammer eingefüllt, diese mit einem Deckel verschlossen und Dibutylphthalat über ein Loch im Deckel mit einer vorgegebenen Dosierrate von 0,0667 ml/s eindosiert. Der Kneter wird mit einer Motordrehzahl von 125 Umdrehungen pro Minute betrieben. Nach Erreichen des Drehmomentmaximums wird der Kneter und die DBP-Dosierung automatisch abgeschaltet. Aus der verbrauchten Menge DBP und der eingewogenen Menge der Partikel wird die DBP-Absorption berechnet nach: DBP-Zahl (g/100 g) = (Verbrauch DBP in g / Einwaage Pulver in g) x 100.

Ein weiterer Gegenstand der Erfindung ist ein Verfahren zur Herstellung des erfindungsgemäßen Silicium-Aluminium-Mischoxidpulvers, bei dem man
a) einen Dampf, der eine oder mehrere Siliciumverbindungen ausgewählt aus der Gruppe bestehend aus CH₃SiCl₃, (CH₃)₂SiCl₂, (CH₃)₃SiCl und (n-C₃H₇)SiCl₃ enthält und den Dampf einer hydrolysierbaren und oxidierbaren Aluminiumverbindung getrennt oder gemeinsam, mittels eines Traggases, in eine Mischkammer überführt, wobei das Gewichtsverhältnis von Aluminiumverbindung, gerechnet als Al₂O₃, zu Siliciumverbindung, gerechnet als SiO₂, 0,003 bis 0,05 ist,
b) getrennt hiervon wenigstens ein Brenngas und Luft in diese Mischkammer überführt, wobei die Gesamtmenge an Sauerstoff in der Luft mindestens ausreichend ist zur vollständigen Verbrennung des Brenngases und der Siliciumverbindungen und Aluminiumverbindungen
c) das Gemisch aus dem Dampf der Siliciumverbindungen und der Aluminiumverbindungen, Brenngas und Luft in einem Brenner zündet und die Flamme in eine Reaktionskammer hinein verbrennt,
d) anschließend den Feststoff von gasförmigen Stoffen abtrennt, und nachfolgend den Feststoff mit Wasserdampf behandelt.

Das Verfahren kann auch so ausgeführt werden, dass der Dampf der Siliciumverbindungen bis zu 40 Gew.-% SiCl₄ enthalten kann. Besonders bevorzugt kann ein Gemisch aus 65 bis 80 Gew.-% CH₃SiCl₃ und 20 bis 35 Gew.-% SiCl₄ sein. Als Aluminiumverbindung eignet sich bevorzugt Aluminiumchlorid. Das Brenngas wird bevorzugt aus der Gruppe bestehend aus Wasserstoff, Methan, Ethan, Propan und Gemischen hiervon ausgewählt. Besonders bevorzugt ist Wasserstoff. Die in die Mischkammer eingebrachte Luft reicht wenigstens zur vollständigen Verbrennung des Brenngases und der Siliciumverbindungen und Aluminiumverbindungen aus. In der Regel wird ein Überschuß an Luft eingesetzt. Die Behandlung mit Wasserdampf dient dem Zweck an den Partikeln anhaftende Chloridreste weitestgehend zu entfernen, so dass das Pulver nicht mehr als 1 Gew.-% Chlorid, bevorzugt nicht mehr als 0,2 Gew.-% Chlorid, enthält.

Darüber hinaus sind die im flammenhydrolytisch hergestellten Oxide bzw. Mischoxide röntgenamorph. Röntgenamorph ist ein Stoff, dessen Fernordnungsreichweite unterhalb der Kohärenzlänge der verwendeten Röntgenstrahlung liegt und somit kein Interferenzmuster erzeugt.

Mit ein Gegenstand der vorliegenden Erfindung sind Mischoxidzusammensetzungen, enthaltend die erfindungsgemäß hergestellten Silicium-Aluminium-Mischoxidpulver, die zusätzlich Alkali- oder Erdalkalimetalloxide aufweisen, wobei die erfindungsgemäßen Mischoxidpulver mit einer wässrigen Alkalimetall- oder Erdalkalimetallhydroxid-Lösung behandelt werden, so dass der pH-Wert im Bereich von 5 bis 6.5 liegt.

Die Alkali- oder Erdalkalimetalle können auf verschiedene Weise eingebracht werden. Es kann zum Beispiel durch Tränken oder Imprägnieren des flammenhydrolytisch hergestellten Mischoxids mit einer Alkali- und/oder Erdalkalimetallsalz-Lösung erfolgen. Eine andere Möglichkeit ist das Mischen des flammenhydrolytisch hergestellten Mischoxids mit einem Alkali- und/oder Erdalkalimetallsalz vor der eigentlichen Herstellung des Katalysators.

Eine weiterer Gegenstand der Erfindung sind Mischoxidzusammensetzungen, enthaltend die erfindungsgemäß hergestellten Silicium-Aluminium-Mischoxidpulver, die zusätzlich mit einer sauren, wässrigen Lösung behandelt werden, wobei der pH-Wert im Bereich von 0 bis 6 liegt.

Es ist vorteilhaft, wenn die Mischoxidzusammensetzung beim Herstellprozess mit einer Phosphorquelle behandelt wird. Als Phosphorquelle kann Phosphorsäure, Phosphonsäure, Phosphinsäure, Polyphosphorsäure oder Dihydrogenphosphat, bevorzugt Phosphorsäure, dienen. Die Behandlung erfolgt so, dass die Mischoxidzusammensetzung in Wasser suspendiert wird und diese Suspension mit der Phosphorquelle versetzt wird, so dass sich ein pH-Wert von 0 bis 6, bevorzugt von 1 bis 2.5, insbesondere von 2 bis 2,5 einstellt. Anschließend wird die der behandelte Katalysator mit Wasser gewaschen, bei 100 bis 150°C getrocknet und bei 300 bis 600°C, bevorzugt bei 450 bis 550°C, kalziniert.

Die Massenanteile der jeweiligen Komponenten der Mischoxidzusammensetzung können folgende Werte annehmen:
a) Silizium: 50 - 99.9 Massen-% (gerechnet als SiO₂),
b) Aluminium: 0.1 - 50 Massen-%, vorzugsweise 0.1 - 20 Massen-%, besonders bevorzugt 1 - 11 Massen-% (gerechnet als Al₂O₃),
c) Alkalimetall: 0 - 15 Massen-% (gerechnet als M₂O) oder Erdalkalimetall: 0 - 30 Massen-% (gerechnet als MO).

Eine weitere Ausführungsform der Erfindung ist dadurch gekennzeichnet, dass aus den erfindungsgemäßen Mischoxidzusammensetzungen unter Zugabe von Bindemitteln, temporären Hilfsstoffen und Fixativen Formkörper in einem formgebenden Verfahren hergestellt werden.

Ebenfalls Gegenstand der vorliegenden Erfindung ist die Verwendung der erfindungsgemäßen Mischoxidzusammensetzungen als Katalysator zur Spaltung einer Ausgangsverbindung der Formel **II** 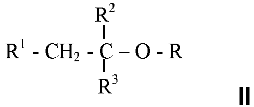 in Isoolefine der Formel **I** 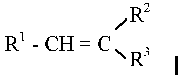 und eine Verbindung der Formel **III**

R - OH **III**

wobei in den Formeln I bis **III** der Rest R für H oder einen Alkylrest mit 1 oder 2 Kohlenstoffatom(en) steht, der Rest R1 für H, Methyl- oder Ethylrest steht und die Reste R2 und R3 für Methyl- oder Ethylreste stehen, wobei die Reste R2 und R3 gleich oder unterschiedlich sein können, bei einer Temperatur von 110 bis 450 °C und einem Druck von 0,1 bis 2 MPa(abs). Ebenfalls Gegenstand der vorliegenden Erfindung ist ein Verfahren zur Spaltung einer Ausgangsverbindung der Formel **II** 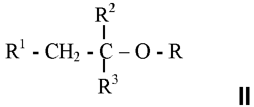 in Isoolefine der Formel **I** 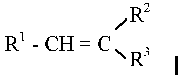 und eine Verbindung der Formel **III**

R - OH **III**

wobei in den Formeln I bis **III** der Rest R für H oder einen Alkylrest mit 1 oder 2 Kohlenstoffatom(en) steht, der Rest R1 für H, Methyl- oder Ethylrest steht und die Reste R2 und R3 für Methyl- oder Ethylreste stehen, wobei die Reste R2 und R3 gleich oder unterschiedlich sein können, bei einer Temperatur 110 bis 450 °C und einem Druck von 0,1 bis 2 MPa(abs), dadurch gekennzeichnet, dass als Katalysator eine flammenhydrolytisch hergestellte Mischoxidzusammensetzung verwendet wird, bei der die Massenanteile der jeweiligen Komponenten der Mischoxidzusammensetzung folgende Werte annehmen können:
a) Silizium: 50 - 99.9 Massen-% (gerechnet als SiO₂),
b) Aluminium: 0.1 - 50 Massen-%, vorzugsweise 0.1 - 20 Massen-%, besonders bevorzugt 1 - 11 Massen-% (gerechnet als Al₂O₃),
c) Alkalimetall: 0 - 15 Massen-% (gerechnet als M₂O) oder Erdalkalimetall: 0 - 30 Massen-% (gerechnet als MO).

In besonderen Ausführungsformen der Erfindung sind zusätzlich Alkali- oder Erdalkalimetalloxide in der als Katalysator für das oben beschriebene Verfahren verwendeten Mischoxidzusammensetzung enthalten, wobei dazu die Mischoxidzusammensetzung mit einer wässrigen Alkalimetall- oder Erdalkalimetallhydroxid-Lösung so behandelt wird, dass bei der Behandlung der pH-Wert im Bereich von 5 bis 6.5 liegt.

Eine weitere Ausführungsform der Erfindung ist dadurch gekennzeichnet, dass die als Katalysator für das oben beschriebene Verfahren verwendete Mischoxidzusammensetzung während ihrer Herstellung mit einer sauren, wässrigen Lösung so behandelt wird, dass bei der Behandlung der pH-Wert im Bereich von 2 bis 2,5 liegt.

Die Herstellung von Isoolefinen durch Gasphasenspaltung von Alkyl-tert.-alkylethern (ATAE) oder tertiären Alkoholen unter Verwendung der erfindungsgemäßen Mischoxidzusammensetzung als Katalysator hat mehrere Vorteile: Auch bei Umsätzen der Einsatzstoffe von über 70 % entstehen die entsprechenden Isoolefine in Selektivitäten von über 99 %. Bei Spaltung von Alkyl-tert.-Butylethern (ATBE) liegen die Selektivitäten für die aus dem abgespalteten Alkohol gebildeten Ethern unter 30 %. Die Spaltung kann bei für Spaltreaktionen relativen niedrigen Temperaturen von 110 bis 450 °C, bevorzugt bei Temperaturen von 180 bis 300 °C durchgeführt werden. Die Umsetzungen kann bei Drücken von größer als 0,3 MPa(abs) durchgeführt werden, sodass eine Kondensation der gebildeten Isoolefine gegen Kühlwasser möglich ist. Der Katalysator zeichnet sich durch eine hohe Standdauer aus. Der Katalysator enthält keine Schwermetalle, sodass weder bei seiner Herstellung noch bei seiner Entsorgung ökologisch bedenkliche Stoffe anfallen. Durch Variation des Anteils an Alkalimetalloxid oder Erdalkalimetalloxid kann die Aktivität für jedes Edukt optimal eingestellt werden.

Das erfindungsgemäße Verfahren zur Herstellung des Katalysators, zeichnet sich dadurch aus, dass ein flammenhydrolytisch hergestelltes Silizium-Aluminium-Mischoxid als Katalysatorkomponente verwendet wird.

Die als Katalysator verwendete erfindungsgemäße Mischoxidzusammensetzung kann neben den Elementen Aluminium, Silizium und Sauerstoff noch Alkali- und/oder Erdalkalimetalle enthalten. Für die industriell interessante Variante der Reaktionsführung in Festbettreaktoren ist es erforderlich, dass die zuvor beschriebene, flammhydrolytisch bzw. pyrogen hergestellte Mischoxidzusammensetzung unter Zugabe eines Bindemittels einem formgebenden Verfahren, wie sie im Stand der Technik hinlänglich bekannt sind, unterworfen wird. Als geeignete Bindemittel können neben Tonerden, keramischen Tonen, Kolloiden beispielsweise auch amorphe Zeolithe Verwendung finden. Zweckmäßigerweise werden die auf diese Weise modifizierten, flammhydrolytisch hergestellten Mischoxidzusammensetzungen in einer Form eingesetzt, in welcher sie einen geringen Strömungswiderstand bieten, wie beispielsweise in Form von Granalien, Pellets oder Formkörpern, wie z.B. Tabletten, Zylindern, Kugeln, Strangextrudaten oder Ringen.
So werden im Allgemeinen 1 - 20 Massen-% der flammhydrolytisch hergestellten Mischoxidzusammensetzung mit einem wie zuvor genannten, trockenen Bindemittel zusammen mit temporären Hilfsstoffen, wie beispielsweise Wasser, wässrigen Lösungen, Wasserersatzstoffen, wie z.B. Glykolen, Polyglykolen und des weiteren Fixativen, wie z.B. Celluloseethern, Plastifizierungsmitteln, wie z.B. Polysacchariden, Presshilfsmitteln, wie z.B. nichtionischen Wachsdispersionen, intensiv vermischt. Dieser Vorgang kann z.B. in einem Kneter oder Intensivmischer erfolgen. Anschließend werden durch ein formgebendes Verfahren, wie beispielsweise der Pelletierung, der Extrusion oder der Trockenpressung, die Formkörper für den Festbettreaktor hergestellt. Vor dem Einbau werden die Formkörper kalziniert in einem Temperaturbereich von 200 - 700°C, wobei die temporären Hilfsstoffe entfernt werden.

In dieser Hinsicht ist eine weitere Ausführungsform der erfindungsgemäßen Zusammensetzung dadurch gekennzeichnet, dass aus dem inerten, porösen Trägermaterial, wie beispielsweise Siliziumdioxid, unter Verwendung eines zusätzlichen Bindemittels, ausgewählt aus der Gruppe umfassend:
a) Tonerde,
b) keramische Tone,
c) Koloide
Formlinge in unterschiedlicher räumlicher Gestalt, ausgewählt aus der Gruppe enthaltend:
i) sphärische,
ii) zylindrische,
iii) ellipsoide,
iv) polilobulare Körper
in einem Größenbereich von 1 - 10 mm gebildet werden.

Der erfindungsgemäße Katalysator weist vorzugsweise eine BET-Oberfläche (volumetrisch bestimmt mit Stickstoff gemäß DIN ISO 9277) von 5 bis 600 m²/g, bevorzugt von 20 bis 200 m²/g auf. Wird der erfindungsgemäße Katalysator als aktive Masse auf einem Träger aufgebracht, so weist nur die aktive Masse eine BET-Oberfläche im genannten Bereich auf. Das Porenvolumen des erfindungsgemäßen Katalysators beträgt vorzugsweise von 0.5 bis 2.0 ml/g, bevorzugt von 0.8 bis 1.5 ml/g. Das Porenvolumen wird vorzugsweise bestimmt durch die Cyclohexanmethode (Bei dieser Methode wird die zu prüfende Probe zunächst bei 110 °C bis zur Gewichtskonstanz getrocknet. Anschließend werden ca. 50 ml der auf 0.01 g genau eingewogenen Probe in ein gereinigtes und bis zur Gewichtskonstanz getrocknetes Imprägnierrohr gefüllt, dass an der Unterseite eine Auslauföffnung mit einem Schliffhahn aufweist. Die Auslauföffnung ist mit einem kleinen Plättchen aus Polyethylen abgedeckt, wodurch ein Verstopfen der Auslauföffnung durch die Probe verhindert wird. Nach dem Befüllen des Imprägnierrohres mit der Probe wird das Rohr sorgfältig luftdicht verschlossen. Anschließend wird das Imprägnierrohr mit einer Wasserstrahlpumpe verbunden, der Schliffhahn geöffnet und über den Wasserstrahl ein Druck im Imprägnierrohr von 20 mbar(absolut) eingestellt. Der Druck kann an einem parallel angeschlossenen Manometer geprüft werden. Nach 20 Min. wird Schliffhahn geschlossen und das evakuierte Imprägnierrohr wird anschließend so mit einer Cyclohexanvorlage, in der ein genau abgemessenes Volumen an Cyclohexan vorgelegt wird, verbunden, dass durch Öffnen des Schliffhahns Cyclohexan aus der Vorlage in das Imprägnierrohr gesaugt wird. Der Schliffhahn bleibt so lange geöffnet, bis die gesamte Probe mit Cyclohexan geflutet ist. Anschließend wird der Schliffhahn wieder verschlossen. Nach 15 min wird das Imprägnierrohr vorsichtig belüftet und das nicht absorbierte Cyclohexan in die Vorlage abgelassen. Im Imprägnierrohr bzw. in der Auslauföffnung oder der Verbindung mit der Cyclohexanvorlage anhaftendes Cyclohexan kann durch einen einzelnen vorsichtigen Druckstoß aus einem Saugball, über die Belüftungsleitung in die Vorlage befördert werden. Das Volumen des in der Vorlage vorhandenen Cyclohexans wird notiert. Das Porenvolumen ergibt sich aus dem absorbierten Cyclohexanvolumen, welches bestimmt wird aus dem Cyclohexanvolumen in der Vorlage vor der Messung minus dem Cyclohexanvolumen in der Vorlage nach der Messung, geteilt durch die Masse der untersuchten Probe).

Der erfindungsgemäße Katalysator kann auch aufgebracht sein auf einem Träger, wie z. B. einen Metall-, Kunststoff- oder Keramikträger, vorzugsweise auf einem in Bezug auf die Reaktion, bei welcher der Katalysator eingesetzt werden soll, inerten Träger. Insbesondere kann der erfindungsgemäße Katalysator auf einen Metallträger, wie z. B. eine Metallplatte oder eine Metallgewebe aufgebracht sein. Solche mit dem erfindungsgemäßen Katalysator ausgerüstete Träger können z. B. als Einbauten in Reaktoren oder Reaktivdestillationskolonnen verwendet werden. Die Träger können auch Metall-, Glas- oder Keramikkugeln oder Kugeln von anorganischen Oxiden sein. Ist der erfindungsgemäße Katalysator auf einem inerten Träger aufgebracht, so wird die Masse und Zusammensetzung des inerten Trägers bei der Bestimmung der Zusammensetzung des Katalysators nicht berücksichtigt.

Der erfindungsgemäße Katalysator kann auch mit inertem Material verdünnt werden. Diese Verdünnung kann durch Mischen des fertigen Katalysators mit dem Inertmaterial vor oder während der Einfüllung des Katalysators in den Reaktor geschehen oder bereits bei der Herstellung des Katalysators erfolgen.

Der erfindungsgemäße Katalysator oder ein mit dem erfindungsgemäßen Verfahren hergestellter Katalysator kann als Katalysator für zahlreiche Reaktionen verwendet werden. Insbesondere kann der erfindungsgemäße Katalysator oder ein mit dem erfindungsgemäßen Verfahren hergestellter Katalysator in einem Verfahren zur Herstellung von Isoolefinen gemäß Formel **I** 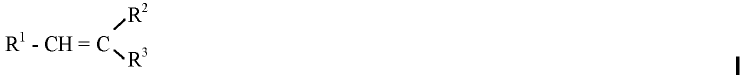 durch katalytische Gasphasenspaltung einer Ausgangsverbindung der Formel **II** 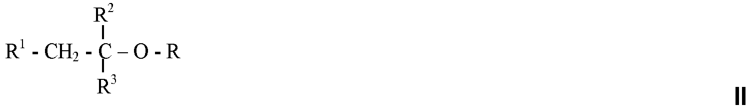 in eine Verbindung der Formel **I** und eine Verbindung der Formel **III**

R - OH **III**

wobei in den Formeln **I** bis **III** der Rest R für H oder einen Alkylrest mit 1 oder 2 Kohlenstoffatom(en) steht, der Rest R¹ für H, Methyl- oder Ethylrest steht und die Reste R² und R³ für Methyl- oder Ethylreste stehen, wobei die Reste R² und R³ gleich oder unterschiedlich sein können, bei einer Temperatur 110 bis 450 °C und einem Druck von 0,1 bis 2 MPa(abs), verwendet werden.

Als Verbindung der Formel **II** können z. B. tertiäre Alkohole mit 4 bis 6-C-Atomen eingesetzt werden. Vorzugsweise wird in einem solchen erfindungsgemäßen Verfahren als Verbindung **II** tert.-Butanol (TBA) in Isobuten als Verbindung der Formel **I** und Wasser als Verbindung **III** gespalten.

Das TBA, welches in dem erfindungsgemäßen Spaltverfahren eingesetzt wird, kann aus verschiedenen technischen Prozessen stammen. Einer der wichtigsten ist die Umsetzung von Isobuten-haltigen C₄-Kohlenwasserstoffgemischen mit Wasser. Verfahren zur Herstellung von TBA werden beispielsweise in den Patentschriften DE 103 30 710 und US 7 002 050 offen gelegt. TBA kann in reiner Form, als TBA/Wasser-Azeotrop oder als sonstiges TBA-Wasser-Gemisch eingesetzt werden.

Bevorzugt wird in einem erfindungsgemäßen Spaltverfahren eine Verbindung der Formel **II**, bei der R ein Methyl-, Ethylrest ist, gespalten. Alkyl-tert.-alkylether die in dem erfindungsgemäßen Spaltverfahren eingesetzt werden können sind beispielsweise Methyl-tert.-butylether, Ethyl-tert.-butylether oder tert.-Amyl-methylether (TAME). Besonders bevorzugt wird bei der erfindungsgemäßen Verwendung des erfindungsgemäßen Katalysators bzw. des erfindungsgemäß hergestellten Katalysators Methyl-tert.-butylether in Isobuten und Methanol oder Ethyl-tert.-butylether in Isobuten und Ethanol gespalten.

Eingesetzt werden können in dem erfindungsgemäßen Spaltverfahren ATAE, die aus den unterschiedlichsten Prozessen stammen können. Ein Verfahren zur Herstellung von MTBE wird beispielsweise in DE 101 02 062 beschrieben. Verfahren zur Herstellung von ETBE werden beispielsweise in DE 10 2005 062700, DE 10 2005 062722, DE 10 2005 062699 oder DE 10 2006 003492 offen gelegt.

Die erfindungsgemäße Spaltung in der Gasphase am erfindungsgemäßen Katalysator wird vorzugsweise bei einer Temperatur von 110 bis 400 °C durchgeführt. Wird als Ausgangsprodukt MTBE eingesetzt, wird die Spaltung von MTBE zu Isobuten und Methanol bevorzugt bei einer Temperatur von 150 bis 350 °C, besonders bevorzugt von 180 bis 300 °C durchgeführt.

Das erfindungsgemäße Spaltverfahren wird vorzugsweise bei einem Reaktionsdruck von 0,1 bis 2 MPa(abs). Wenn Isobuten ein Produkt ist kann es vorteilhaft sein, das erfindungsgemäße Spaltverfahren bei einem Druck von 0,2 bis 1 MPa(abs), bevorzugt von 0,5 bis 0,8 MPa(abs) durchzuführen. Dies ist insbesondere deshalb vorteilhaft, weil bei diesen Drücken Isobuten gegen Kühlwasser kondensiert werden kann.

Die spezifische Katalysatorbelastung (WHSV; Gramm Edukt bei Raumtemperatur je Gramm Katalysator je Stunde) beträgt im erfindungsgemäßen Spaltverfahren vorzugsweise von 0,1 bis 100 h⁻¹, bevorzugt von 0,5 bis 30 h⁻¹. Wird als Ausgangsprodukt MTBE eingesetzt, wird die Spaltung von MTBE zu Isobuten und Methanol bevorzugt bei einer WHSV von 0,1 bis 100 h⁻¹, besonders bevorzugt von 0,25 bis 25 h⁻¹ durchgeführt.

Um den Aufarbeitungsaufwand des Spaltproduktgemisches gering zu halten, werden vorzugsweise hohe Umsätze für den geraden Durchgang angestrebt. Vorzugsweise wird das erfindungsgemäße Verfahren so durchgeführt, dass die Umsätze an zu spaltender Verbindung über 70 %, bevorzugt über 80 % und besonders bevorzugt von über 90 % bis 100 % betragen. Wenn die Edukte störende Nebenkomponenten enthalten, kann es zweckmäßig sein, den Umsatz zu begrenzen. Enthält beispielsweise das Einsatzstoffgemisch neben dem zu spaltenden MTBE auch 2-Methoxybutan, kann es notwendig sein, den Umsatz im geraden Durchgang zu verringern, um ein vorgegebenes Verhältnis von linearen Butenen zu Isobuten im Reaktionsgemisch nicht zu überschreiten. Somit kann es vorteilhaft sein, den zulässigen Umsatz an MTBE zu begrenzen, je höher der Anteil an 2-Methoxybutan im MTBE aufweisenden Einsatzstoffgemisch ist. Die Begrenzung des Umsatzes kann beispielsweise durch Erhöhung der WHSV und/oder Absenken der Reaktionstemperatur erreicht werden.

Es kann für die Aktivität und/oder Selektivität einiger Katalysatoren vorteilhaft sein, wenn dem Reaktorzulauf Anteile an Wasser zugegeben werden. So beschreibt beispielsweise JP 19912201 die kontinuierliche Zugabe von Wasser zu einem Alkali- oder Erdalkalimetall-dotiertem Alumosilikat zur Verringerung der Bildung von Nebenkomponenten.

Die optionale Zugabe von Wasser zur Moderation des Katalysators erfolgt derart, dass der Wasseranteil im Reaktoreingang bevorzugt 0 bis 5 Massen-%, besonders bevorzugt 0,2 bis 1,5 Massen-% beträgt. Als zugeführtes Wasser wird dabei bevorzugt vollständig demineralisiertes oder destilliertes Wasser oder Wasserdampf eingesetzt.

Das Spaltproduktgemisch kann nach bekannten technischen Verfahren aufgearbeitet werden. Nicht umgesetztes Edukt kann gegebenenfalls nach einer Teilausschleusung oder Reinigung in die Spaltung zurückgeführt werden.

Die gewonnenen Isoolefine können, wie in der Einleitung beschrieben, genutzt werden.

Ein gemäß dem erfindungsgemäßen Spaltverfahren hergestelltes Isobuten kann insbesondere zur Herstellung von Butylkautschuk, Polyisobutylen, Isobuten-Oligomeren, verzweigten C₅-Aldehyden, C₅-Carbonsäuren, C₅-Alkoholen, C₅-Olefinen, tert.-Butylaromaten und Methacrylsäure und deren Ester eingesetzt werden.

Die bei der Spaltung von ATAE anfallenden Alkohole können nach Aufarbeitung wieder verwendet werden, beispielsweise zur Synthese von ATAE.

Das erfindungsgemäße Verfahren sowie die erfindungsgemäßen Katalysatoren werden nachfolgend beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche oder Teilgruppen von Verbindungen, die durch Herausnahme von einzelnen Werten (Bereichen) oder Verbindungen erhalten werden können.

Die folgenden Beispiele sollen den erfindungsgemäßen Katalysator und das Verfahren unter Verwendung des erfindungsgemäßen Katalysators erläutern.

### Beispiel 1: Herstellung des erfindungsgemäßen Silicium-Aluminium-Mischoxidpulvers

Der Dampf eines Gemisches bestehend aus 45 kg/h CH₃SiCl₃ und 15 kg/h SiCl₄ und der Dampf 0,6 kg/h Aluminiumchlorid werden getrennt voneinander mittels Stickstoff als Traggas in eine Mischkammer überführt. Die Dämpfe werden mit 14,6 Nm³/h Wasserstoff und 129 Nm³/h getrockneter Luft in der Mischkammer eines Brenners gemischt, und über ein Zentralrohr, an dessen Ende das Reaktionsgemisch gezündet wird, einem wassergekühlten Flammrohr zugeführt und dort verbrannt. Das entstandene Pulver wird anschließend in einem Filter abgeschieden und mit Wasserdampf bei 400 - 700°C behandelt. Das Pulver enthält 99 Gew.-% Siliciumdioxid und 1 Gew.-% Aluminiumoxid. Die BET-Oberfläche beträgt 173 m²/g. Die DBP-Zahl beträgt 326 g / 100 g Mischoxid.

Zur Bestimmung des Gewichtsverhältnises (Al₂O₃/SiO₂)_{Oberfläche} der Primärpartikel in einer oberflächennahen Schicht mit einer Dicke von ca. 5 nm wird eine XPS-Analyse angewandt. Sie liefert ein Gewichtsverhältnis (Al₂O₃/SiO₂)_{Oberflache} von 0,0042. Die Bestimmung des Gewichtsverhältnises (Al₂O₃/SiO₂)ₜₜₗ im Gesamtprimärpartikel erfolgt durch Röntgenfluoreszenzanalyse am Pulver. Sie liefert ein Gewichtsverhältnis (Al₂O₃/SiO₂)_{ttl.}von 0,010. Damit ergibt sich ein Wert für (Al₂O₃/SiO₂)ₜₜₗ/ (Al₂O₃/SiO₂)_{oberfläche} von 2,4.

### Beispiel 2: Herstellung von Katalysator-Extrudaten (erfindungsgemäß)

600 g pyrogen hergestelltes Alumosilikat (1 Massen-% Al, gerechnet als Al₂O₃), 24 g eines handelsüblichen Celluloseethers, 21 g einer handelsüblichen nichtionischen Wachsdispersion als Presshilfsmittel, 3 g eines handelsüblichen Polysaccharids als Plastifizierungsmittel, 6 g 30 %ige wässrige NH₃-Lösung und voll entsalztes Wasser werden in einem Intensivmischer mit Stiftwirbler vermengt. Anschließend erfolgt eine Pelletierung in dem Intensivmischer, bei der innerhalb von 30-40 Minuten gleichmäßig rundliche Pellets mit einem Durchmesser von ca. 1 bis 3 mm erhalten werden. Die feuchten Pellets werden mit einem handelsüblichen Extruder zu 3 mm langen Extrudaten verarbeitet (Schneckengehäuse 300 mm, Schneckendurchmesser 80-64 mm, Getriebedrehgeschwindigkeit 160 U/min, Extrusionsdruck 31 kg/h). Die so erhaltenen Extrudate werden bei 120°C im Luftstrom getrocknet und bei 600°C an Luft kalziniert.

### Beispiel 3: Herstellung von Natrium-dotiertem Katalysatorpulver (erfindungsgemäß)

10 g pyrogenes Alumosilikat (1 Massen-% Al, gerechnet als Al₂O₃) und 400 ml destilliertes Wasser werden in einen 500 ml Doppelmantel-Reaktor aus Glas überführt. Gleichzeitig befindet sich ein Liebigkühler zentral auf dem Reaktor, um das Verdampfen und Austreten der Flüssigphase zu vermeiden. Der pH-Wert der Suspension (ca. 4) wird durch Zugabe von NaOH auf den gewünschten Wert (pH = 5, 6, oder 6.5) erhöht. Die Suspension wird mit Hilfe eines Magnetrührers über die gesamte Reaktionszeit gerührt und über einen angeschlossen Thermostaten auf 70°C geheizt. Nach 20h wird die Suspension auf Umgebungstemperatur abgekühlt und filtriert. Der hieraus gewonnene Feststoff wird anschließend bei einer Rate von 1°C/min auf 500°C in einem Muffelofen geheizt und 5h bei Endtemperatur kalziniert.

### Beispiel 4: Herstellung von Phosphorsäure-behandeltem Katalysatorpulver (erfindungsgemäß)

10 g pyrogenes Alumosilikat (1 Massen-% Al, gerechnet als Al₂O₃) werden in 400 ml destilliertem Wasser verrührt (pH ca. 4). 85%ige Phosphorsäure wird der Suspension zugegeben bis der gewünschte pH-Wert erreicht wird (pH = 2.5 bzw. pH = 2.0). Die Lösung wird 2 h bei Raumtemperatur gerührt, anschließend wird der Feststoff abfiltriert und bei 120°C für 1h getrocknet (Aufheizrate = 1°C/min). Anschließend wird der hieraus gewonnene Feststoff mit 500 ml destilliertem Wasser gewaschen, filtriert und bei 500°C für 5h kalziniert (Aufheizrate = 1°C/min).

### Beispiel 5: Gasphasenspaltung von Methyl-tert.-butylehter (MTBE) zu Isobuten und Methanol sowie tert.-Butylalkohol (TBA) zu Isobuten und Wasser

Die Reaktionen wurden in einer vollautomatisierten Katalysatortest-Apparatur ausgestattet mit 12 parallelen Reaktoren (800 x 8mm) isotherm bei T = 498 K and P = 0,6 MPa(abs) durchgeführt. Zur Erhöhung der statistischen Sicherheit wurden jeweils zwei Proben der Katalysatoren (Einwaage: 0.2g als Pulver) parallel getestet. Die Katalysator-Proben wurden im Verhältnis 1:5 mit granuliertem Quartz gemischt, um eine isothermes Temperaturverhalten im Katalysatorbett zu gewährleisten. Als Referenzkatalysator wird ein mit 10 Massen-% Magnesium (gerechnet als MgO) dotiertes Alumosilikat mit 21 Massen-% Aluminiumgehalt (gerechnet als Al₂O₃), kommerziell erhältlich als Specialyst 071 der Evonik Degussa GmbH, verwendet. MTBE wird flüssig über einen Massendurchflussregler dosiert und in einem, den parallelen Reaktoren vorgeschalteten Verdampfer-Rohr (200 x 24mm) gefüllt mit Glaskugeln (d = 2 mm) in die Gasphase überführt. Nach dem Verdampfer wird der Gasstrom in einem Verteiler in 12 gleiche Anteile geteilt. Dies wird durch nachgeschaltete Restriktoren sichergestellt. Jeder Gasstromanteil wird durch einen der 12 parallelen Reaktoren geleitet. Das die Reaktoren verlassende Abgas wird nacheinander über ein Auswahlventil zu einem on-line Gaschromatographen geleitet und analysiert. Die übrigen 11 Ströme werden gesammelt und der Entsorgung zugeführt.

Die spezifische Katalysatorbelastung (WHSV; Gramm Edukt je Gramm Katalysator je Stunde) wurde zwischen 5 and 50 h⁻¹ variiert.

**Tabelle 1: Spaltung von MTBE**

| Reaktionsbedingungen: 225°C, 0,6 MPa(abs), 0.2g Katalysator; Ergebnisse wurder nach 100h on stream erhalten. | | | | |
|---|---|---|---|---|
| Katalysator | WHSV [h⁻¹] | MTBE-Umsatz [%] | DME-Selektivität [%] | C₈-Selektivität [%] |
| **A**: nicht erfindungsgem. Kat. mit 11 Massen-% Al | 18 | 85 | 1.16 | 0.03 |
| **B**: nicht erfindungsgem. Kat. mit 1 Massen-% Al | 18 | 85 | 0.63 | 0.06 |
| **C**: Kat. nicht gem. Erfindung | 8 | 85 | 2.29 | 0.10 |
| **D:** nicht erfindungsgem.Kat. mit 50 Massen-% Al | 25 | 85 | 1.49 | 0.17 |
| **E:** nicht erfindungsgem.Kat. mit 0.1 Massen-% Al und BET = 20 m²/g | 8 | 35 | 0.07 | 0.01 |
| **F:** erfindungsgem.Kat. aus Beispiel 2 (pH 5) | 17 | 85 | 0.35 | 0.08 |
| **G:** erfindungsgem.Kat. aus Beispiel 2 (pH 6) | 16 | 85 | 0.37 | 0.06 |
| **H:** erfindungsgem.Kat. aus Beispiel 2 (pH 6.5) | 5 | 85 | 0.49 | 0.05 |
| **I:** nicht erfindungsgem.Kat. aus Beispiel 3 (pH 2) | 35 | 85 | 0.13 | 0.09 |
| **J:** nicht erfindungsgem.Kat. aus Beispiel 3 (pH 2.5) | 28 | 85 | 0.24 | 0.08 |
| **K:** nicht erfindungsgem.Kat. mit 1 Massen-% Al und BET = 80 m²/g | 8 | 85 | 0.92 | 0.05 |

| | | | | |
|---|---|---|---|---|
| *Al-Gehalt jeweils gerechnet als Al₂O₃ A: pyrogenes Al/Si 11/ 200 m²/g BET-Oberfläche B: pyrogenes Al/Si 1/ 200 m²/g BET-Oberfläche C: Specialyst 071 (gefälltes Alumosilikat mit 21 % Al₂O₃, dotiert mit 10% MgO) Produkt der Evonik Degussa GmbH D: pyrogenes Al/Si 50/ 50 m²/g BET-Oberfläche E: pyrogenes Al/Si 0.1/ 20 m²/g BET-Oberfläche F: pyrogenes Al/Si wie B, behandelt mit NaOH gemäß Beispiel 3 (pH 5) G: pyrogenes Al/Si wie B, behandelt mit NaOH gemäß Beispiel 3 (pH 6) H: pyrogenes Al/Si wie B, behandelt mit NaOH gemäß Beispiel 3 (pH 6.5) I: pyrogenes Al/Si wie B, behandelt mit H₃PO₄ gemäß Beispiel 4 (pH 2) J: pyrogenes Al/Si wie B, behandelt mit H₃PO₄ gemäß Beispiel 4 (pH 2.5) K: pyrogenes Al/Si, 80 m²/g BET-Oberfläche) | | | | |

**Tabelle 2: Spaltung von TBA**

| Reaktionsbedingungen: 225°C, 0,6 MPa(abs), 0.2g Katalysator; Ergebnisse wurden nach 100h on stream erhalten. | | | | |
|---|---|---|---|---|
| Katalysator | WHSV [h⁻¹] | TBA-Umsatz [%] | C₈-Selektivität [%] | C₁₂-Selektivität [%] |
| Kat. nicht gem. Erfindung (Specialyst 071) | 75 | 80 | 0.11 | 0.05 |
| Kat. gem. Erfindung mit 1 Massen-% Al | 75 | 99 | 0.01 | 0.01 |

| | | | | |
|---|---|---|---|---|
| *Al-Gehalt jeweils gerechnet als Al₂O₃ | | | | |

### Beispiel 6: Langzeitversuch Gasphasenspaltung von Methyl-tert.-butylehter (MTBE) zu Isobuten und Methanol

Die Spaltung wurde in einem Festbettreaktor mit Heizmantel, durch den ein Wärmeträgeröl (Marlotherm SH der Sasol Olefins & Surfactants GmbH) floss, durchgeführt. Als Edukt wurde MTBE technischer Qualität (Diveron der Fa. Evonik Oxeno GmbH) mit einer Reinheit von 99.7 Massen-% eingesetzt.

Vor dem Eintritt in den Reaktor wurde das MTBE in einem Verdampfer bei 180 - 270°C vollständig verdampft. Bei einer Temperatur von 180 - 270°C (Temperatur des Marlotherms im Zulauf des Reaktormantels) und einem Druck von 0,6 MPa(abs) wurden stündlich 1500 g MTBE durch 300 g Katalysator, entsprechend einem WHSV-Wert von 5 h⁻¹, durchgeleitet. Das gasförmige Produktgemisch wurde gaschromatographisch analysiert. Zum Ausgleich der fortlaufenden Katalysatordesaktivierung wurde die Temperatur kontinuierlich erhöht, so dass stets ein Umsatz von 85% erzielt wurde.

Als Katalysatoren werden der erfindungsgemäße Katalysator aus Beispiel 1 in Form von 3 mm-großen zylindrischen Extrudaten sowie der nicht erfindungsgemäße kommerzielle Katalysator Specialyst 071 der Evonik Degussa GmbH (gefälltes Alumosilikat mit 21% Al₂O₃, dotiert mit 10% MgO) in Form von 3 mm-großen zylindrischen Tabletten eingesetzt. Aufgrund der hohen katalytischen Aktivität der erfindungsgemäßen Katalysatoren, wird dieser mit der 2-fachen Masse Inertmaterial , wie z.B. einem handelsüblichen α-Al₂O₃ wie Spheralite 512, verdünnt, um eine gleiche WHSV einstellen zu können.

Aus der Zusammensetzung von Edukt- und Produktgemisch wurden die Isobuten-Umsätze, die Selektivitäten der Dimethylether-Bildung (DME-Selektivität = 2 * Molzahl des gebildeten DME zu Molzahl des umgesetzten MTBE) und die Selektivitäten der Okten-Bildung (C₈-Selektivität = 2 * Molzahl des gebildeten Oktens zu Molzahl des umgesetzten MTBE) bei verschiedenen Reaktionszeiten berechnet. Diese Werte wurden in den nachfolgenden Tabellen 3 und 4 zusammengestellt.

**Tabelle 3: Umsätze. Selektivitäten und Temperaturen der Spaltung von MTBE mit dem in Beispiel 1 hergestellten Katalysator (verdünnt mit der 2-fachen Masse Inertmaterial)**

| | | | | | | |
|---|---|---|---|---|---|---|
| Versuchsdauer [h] | 200 | 400 | 600 | 1000 | 1400 | 1600 |
| Temperatur [°C] | 220 | 230 | 240 | 245 | 248 | 248 |
| MTBE-Umsatz [%] | 85 | 85 | 85 | 85 | 85 | 85 |
| DME-Selektivität [%] | 0.15 | 0.3 | 0.4 | 0.6 | 0.7 | 0.7 |
| C₈-Selektivität [%] | 0.3 | 0.17 | 0.09 | 0.08 | 0.07 | 0.07 |

**Tabelle 4: Umsätze, Selektivitäten und Temperaturen der Spaltung von MTBE mit dem Vergleichs-Katalysator Specialyst 071**

| | | | | | | |
|---|---|---|---|---|---|---|
| Versuchsdauer **[**h**]** | 100 | 250 | 500 | 750 | 1000 | 1500 |
| Temperatur [°C] | 240 | 252 | 258 | 259 | 261 | 262 |
| MTBE-Umsatz **[%]** | 85 | 85 | 85 | 85 | 85 | 85 |
| DME-Selektivität **[%]** | 1.6 | 2.4 | 2.9 | 3.2 | 3.4 | 3.7 |
| C₈-Selektivität **[%]** | 0.19 | 0.12 | 0.08 | 0.08 | 0.07 | 0.06 |

### Interpretation der Versuchsergebnisse

Tabelle 1 in Beispiel 5 zeigt die Versuchsergebnisse der MTBE-Spaltung mit den erfindungsgemäßen Katalysatoren in Pulverform im Vergleich zum nicht erfindungsgemäßen kommerziell erhältlichen Katalysator Specialyst 071 (ebenfalls als Pulver). Darüber hinaus wurden erfindungsgemäße Katalysatoren mit unterschiedlichen Aluminium-Gehalten sowie unterschiedlichen BET-Oberflächen -20, 50, 80 und 200 m²/g - verglichen.

Man kann erkennen, dass der nicht erfindungsgemäße Katalysator **C** mit einer DME-Selektivität von 2.29% den höchsten Wert aller getesteten Katalysatoren aufweist. Die katalytische Aktivität ist ebenfalls relativ gering, da eine niedrige WHSV von 8 h⁻¹ eingestellt werden muss, um den Umsatz von 85% zu erreichen. Die C₈-Selektivität ist ebenfalls mit 0.1% recht hoch.

Im Vergleich dazu zeigt der erfindungsgemäße Katalysator **B** mit 1 Massen-% Aluminiumgehalt die besten Ergebnisse. Der Umsatz von 85% wird bereits mit einer WHSV von 18 h⁻¹ erreicht. Gleichzeitig sind die Selektivitäten zu DME und C₈ mit Werten von 0.63% bzw. 0.06% sehr niedrig.

Der Katalysator **E** mit 0.1 Massen-% Aluminiumgehalt und einer BET-Oberfläche von 20 m²/g zeigt noch niedrigere Selektivitäten zu den Nebenprodukten, kommt jedoch aufgrund seiner geringen katalytischen Aktivität nur zu einem Umsatz von 35% bei einer WHSV von 8⁻¹.

Der erfindungsgemäße Katalysator **A** mit 11 Massen-% Aluminiumgehalt zeigt ebenfalls niedrigere Nebenprodukt-Selektivitäten und eine höhere katalytische Aktivität als der nicht erfindungsgemäße Referenzkatalysator. Bei der DME-Selektivität ist er mit einem Wert von 1.16% etwas schlechter als der erfindungsgemäße Katalysator **B** mit 1 Massen-% Al-Gehalt, bei der C₈-Selektivität hingegen ist er mit 0.03% leicht besser. Der Katalysator **D** mit einem Aluminiumgehalt von 50 Massen-% zeigt die höchste Aktivität aller getesteten Katalysatoren, jedoch zeigt er auch relativ hohe Nebenproduktselektivtäten.

Die mit NaOH dotierten Katalysatoren **F, G,** oder **H** mit 1 Massen-% Aluminiumgehalt aus Beispiel 3 zeigen abhängig vom eingestellten pH-Wert eine leicht geringere Aktivität als der entsprechende undotierte Katalysator **B.** Sie zeigen jedoch vergleichsweise noch geringere Nebenproduktselektivitäten hinsichtlich DME- bzw. C₈-Bildung.

Die mit Phosphorsäure moderierten Katalysatoren **I** oder **J** mit 1 Massen-% Aluminiumgehalt aus Beispiel 4 zeigen abhängig vom eingestellten pH-Wert eine höhere Aktivität als der entsprechende unmoderierte Katalysator **B.** Gleichzeitig ist eine noch niedrigere DME-Bildung zu beobachten, während die C₈-Bildung nur leicht erhöht ist.

Im Vergleich zum Katalysator **B** mit 1 Massen-% Aluminiumgehalt und einer BET-Oberfläche von 200 m²/g zeigt Katalysator **K** mit einer BET-Oberfläche von nur 80 m²/g eine nierdrigere katalytische Aktivität. Die Selektivitäten zu den Nebenprodukten sind auf einem ähnlichen Niveau.

Zusammenfassend lässt sich aus diesen Ergebnissen ableiten, dass die neuen erfindungsgemäßen Katalysatoren eine höhere katalytische Aktivität sowie eine geringere Bildung der unerwünschten Nebenprodukte Dimethylether (DME) und 2,4,4-Trimethylpentenen (C₈) zeigen. Des Weiteren kann man erkennen, dass ein niedriger Aluminiumgehalt bei den erfindungsgemäßen Katalysatoren, wie z.B. Katalysator E, zu einer geringeren Nebenproduktbildung, aber auch zu einer geringeren katalytischen Aktivität führt. Der gleiche Trend ist bei der Erhöhung des Natriumgehaltes festzustellen.

Tabelle 2 in Beispiel 5 zeigt die Ergebnisse der Spaltung von tert.-Butanol (TBA) mit dem erfindungsgemäßen Katalysator **B** mit einem Aluminiumgehalt von 1% im Vergleich zum nicht erfindungsgemäßen, kommerziell erhältlichen Katalysator **C,** Specialyst 071, jeweils in Pulverform. Hierbei ist zu erkennen, dass der erfindungemäße Katalysator sowohl eine höhere katalytische Aktivität als auch eine geringere Bildung der unerwünschten Nebenprodukte 2,4,4-Trimethylpentene (C₈) und C₁₂-Komponenten zeigt.

Tabellen 3 und 4 in Beispiel 6 zeigen die Ergebnisse der Langzeitversuche in der MTBE-Spaltung mit dem erfindungsgemäßen Katalysator **B** mit einem Aluminiumgehalt von 1 Massen-% und dem nicht erfindungsgemäßen Katalysator **C,** Specialyst 071, aus einer Pilotanlage. Beide Katalysatoren wurden hier nicht als Pulver, sondern als Formkörper eingesetzt. Aufgrund der hohen katalytischen Aktivität wird der erfindungsgemäße Katalysator **B** mit der 2-fachen Masse an Inertmaterial (a-Aluminiumoxid) verdünnt.

Man kann bei beiden Versuchen erkennen, dass aufgrund der langsamen Desaktivierung die Temperatur innerhalb der ersten ca. 1500 h um 20-30°C angehoben werden muss. Beim erfindungsgemäßen Katalysator **B** findet dies jedoch im Vergleich zum Referenzkatalysator **C** auf einem niedrigeren Temperaturniveau statt, so dass die apparative Temperaturobergrenze erst später erreicht wird.

In den meisten Fällen wird im technischen Betrieb die Katalysatorlebensdauer bei der Spaltung von Alkyl-tert.-alkylethern und tert.-Alkoholen mit dem Ziel der Herstellung von hochreinen Isoolefinen jedoch nicht durch die Aktvität des Katalysators beschränkt, sondern durch die Produktion von unerwünschten Nebenprodukten. Ab einer bestimmten Menge an Nebenprodukten ist die technische Anlage nicht mehr in der Lage die Nebenprodukte soweit abzutrennen, dass die gewünschten Produktspezifikationen erreicht werden. Hier ist der erfindungsgemäße Katalysator **B** stark im Vorteil, da er weniger Nebenprodukte bildet. Insbesondere das schwer abzutrennende DME wird zu einem geringeren Maße gebildet.

## Patentansprüche

1. Mischoxidzusammensetzung, enthaltend ein Silicium-Aluminium-Mischoxidpulver, welches überwiegend oder vollständig in Form von aggregierten Primärpartikeln vorliegt und bei dem
a) das Gewichtsverhältnis von (Al₂O₃/SiO₂)ₜₜₗ im Gesamtprimärpartikel 0,002 bis 0,05, bevorzugt 0,003 bis 0,015, besonders bevorzugt 0,005 bis 0,01,
b) das Gewichtsverhältnis (Al₂O₃/SiO₂)_{Oberfläche} der Primärpartikel in einer oberflächennahen Schicht mit einer Dicke von 5 nm kleiner als im Gesamtprimärpartikel und
c) die BET-Oberfläche 50 bis 250 m²/g, bevorzugt 100 bis 200 m²/g, ist; sowie Alkali- oder Erdalkalimetalloxide.

2. Mischoxidzusammensetzung nach Anspruch 1 erhältlich durch Behandeln mit einer wässrigen Alkali- oder Erdalkalimetallhydroxid-Lösung, wobei der pH-Wert in einem Bereich von 5 bis 6,5 liegt.

3. Mischoxidzusammensetzung nach einem der vorherigen Ansprüche, **dadurch gekennzeichnet, dass** unter Zugabe von Bindemitteln, temporären Hilfsstoffen und Fixativen Formkörper in einem formgebenden Verfahren hergestellt werden.

4. Verwendung einer Mischoxidzusammensetzung nach einem der vorherigen Ansprüche als Katalysator zur Spaltung einer Ausgangsverbindung der Formel **II** 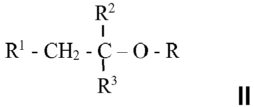 in Isoolefine der Formel **I** 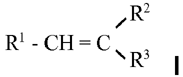 und eine Verbindung der Formel **III**
R - OH **III**
wobei in den Formeln **I** bis **III** der Rest R für H oder einen Alkylrest mit 1 oder 2 Kohlenstoffatom(en) steht, der Rest R1 für H, Methyl- oder Ethylrest steht und die Reste R2 und R3 für Methyl- oder Ethylreste stehen, wobei die Reste R2 und R3 gleich oder unterschiedlich sein können.

5. Verfahren zur Spaltung einer Ausgangsverbindung der Formel **II** 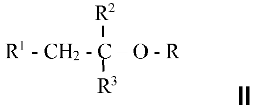 in Isoolefine der Formel **I** 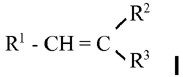 und eine Verbindung der Formel **III**
R - OH **III**
wobei in den Formeln **I** bis **III** der Rest R für H oder einen Alkylrest mit 1 oder 2 Kohlenstoffatom(en) steht, der Rest R1 für H, Methyl- oder Ethylrest steht und die Reste R2 und R3 für Methyl- oder Ethylreste stehen, wobei die Reste R2 und R3 gleich oder unterschiedlich sein können,
**dadurch gekennzeichnet, dass** eine Mischoxidzusammensetzung als Katalysator nach den vorherigen Ansprüchen eingesetzt wird.

6. Verfahren nach Anspruch 5, **dadurch gekennzeichnet, dass** eine flammenhydrolytisch hergestellt Mischoxidzusammensetzung als Katalysator eingesetzt wird, der die folgende Zusammensetzung hat:
a. Silizium: 50 - 99.9 Massen-% (gerechnet als SiO₂),
b. Aluminium: 0.1 - 50 Massen-%, vorzugsweise 0.1 - 20 Massen-%, besonders bevorzugt 1 - 11 Massen-% (gerechnet als Al₂O₃),
c. Alkalimetall: 0 - 15 Massen-% (gerechnet als M₂O) oder Erdalkalimetall: 0 - 30 Massen-% (gerechnet als MO).

7. Verfahren nach Anspruch 5 oder 6, wobei Methyl-tert.-butylether als Ausgangsmaterial verwendet wird.

8. Verfahren nach Anspruch 5 oder 6, wobei tert.-Butylalkohol als Ausgangsmaterial verwendet wird.

9. Verfahren nach Anspruch 8, wobei tert.-Butanol in azotroper Mischung mit Wasser als Ausgangsmaterial verwendet wird.

10. Verfahren nach Anspruch 5 oder 6, wobei die Temperatur in einem Bereich von 110 bis 450 °C liegt.

11. Verfahren nach Anspruch 5 oder 6, wobei der Druck in einem Bereich von 0,1 bis 2 MPa(abs) liegt.

## Claims

1. Mixed oxide composition comprising a silicon-aluminium mixed oxide powder which is present predominantly or completely in the form of aggregated primary particles and in which
a) the weight ratio (Al₂O₃/SiO₂)ₜₜₗ in the overall primary particle is 0.002 to 0.05, preferably 0.003 to 0.015, more preferably 0.005 to 0.01,
b) the weight ratio (Al₂O₃/SiO₂) _{surface} of the primary particles in a layer close to the surface having a thickness of 5 nm is less than in the overall primary particle and
c) the BET surface area is 50 to 250 m²/g, preferably 100 to 200 m²/g; and alkali metal or alkaline earth metal oxides.

2. Mixed oxide composition according to Claim 1, obtainable by treating with an aqueous alkali metal or alkaline earth metal hydroxide solution, where the pH is within a range from 5 to 6.5.

3. Mixed oxide composition according to any one of the preceding claims, **characterized in that** binders, temporary assistants and fixatives are added to produce shaped bodies in a shaping process.

4. Use of a mixed oxide composition according to any of the preceding claims as a catalyst for cleaving a starting compound of the formula **II** 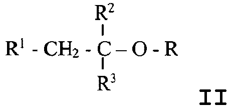 to isoolefins of the formula **I** 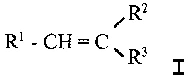 and a compound of the formula **III**
R - OH **III**
where the R radical in the formulae **I** to **III** is H or an alkyl radical having one or two carbon atom(s), the R1 radical is H, or a methyl or ethyl radical and the R2 and R3 radicals are each methyl or ethyl radicals, where the R2 and R3 radicals may be the same or different.

5. Process for cleaving a starting compound of the formula **II** 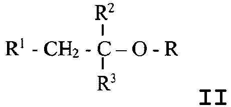 to isoolefins of the formula **I** 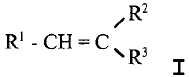 and a compound of the formula **III**
R - OH **III**
where the R radical in the formulae **I** to **III** is H or an alkyl radical having one or two carbon atom(s), the R1 radical is H, or a methyl or ethyl radical and the R2 and R3 radicals are each methyl or ethyl radicals, where the R2 and R3 radicals may be the same or different, **characterized in that** a mixed oxide composition according to the preceding claims is used as a catalyst.

6. Process according to Claim 5, **characterized in that** the catalyst used is a mixed oxide composition which is produced by flame hydrolysis and has the following composition:
a. silicon: 50 - 99.9% by mass (calculated as SiO₂) ,
b. aluminium: 0.1 - 50% by mass, preferably 0.1 - 20% by mass, more preferably 1 - 11% by mass (calculated as Al₂O₃),
c. alkali metal: 0 - 15% by mass (calculated as M₂O) or alkaline earth metal: 0 - 30% by mass (calculated as MO).

7. Process according to Claim 5 or 6, wherein methyl tert-butyl ether is used as the starting material.

8. Process according to Claim 5 or 6, wherein tert-butyl alcohol is used as the starting material.

9. Process according to Claim 8, wherein tert-butanol in an azeotropic mixture with water is used as the starting material.

10. Process according to Claim 5 or 6, wherein the temperature is within a range from 110 to 450°C.

11. Process according to Claim 5 or 6, wherein the pressure is within a range from 0.1 to 2 MPa(abs).

## Revendications

1. Composition d'oxyde mixte, contenant une poudre d'oxyde mixte de silicium-aluminium, qui se présente principalement ou entièrement sous la forme de particules primaires agrégées, et dans laquelle
a) le rapport en poids (Al₂O₃/SiO₂)ₜₜₗ dans les particules primaires dans leur ensemble est de 0,002 à 0,05, de préférence de 0,003 à 0,015, de manière particulièrement préférée de 0,005 à 0,01,
b) le rapport en poids (Al₂O₃/SiO₂)surface des particules primaires dans une couche proche de la surface d'une épaisseur de 5 nm est inférieur à celui dans les particules primaires dans leur ensemble, et
c) la surface BET est de 50 à 250 m²/g, de préférence de 100 à 200 m²/g ;
ainsi que des oxydes de métaux alcalins ou alcalino-terreux.

2. Composition d'oxyde mixte selon la revendication 1, pouvant être obtenue par traitement avec une solution aqueuse d'hydroxyde de métal alcalin ou alcalino-terreux, le pH se situant dans une plage allant de 5 à 6,5.

3. Composition d'oxyde mixte selon l'une quelconque des revendications précédentes, **caractérisée en ce que** des corps moulés sont fabriqués avec ajout de liants, d'adjuvants temporaires et d'agents fixateurs par un procédé de façonnage.

4. Utilisation d'une composition d'oxyde mixte selon l'une quelconque des revendications précédentes en tant que catalyseur pour le clivage d'un composé de départ de formule II 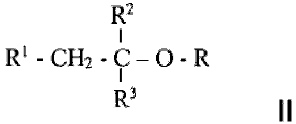 en isooléfines de formule I 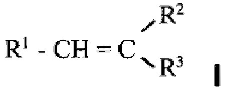 et un composé de formule III
R-OH III
dans les formules I à III, le radical R représentant H ou un radical alkyle contenant 1 ou 2 atomes de carbone, le radical R1 représentant H, un radical méthyle ou éthyle, et les radicaux R2 et R3 représentant des radicaux méthyle ou éthyle, les radicaux R2 et R3 pouvant être identiques ou différents.

5. Procédé de clivage d'un composé de départ de formule II 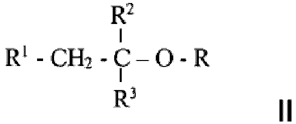 en isooléfines de formule I 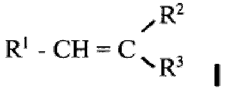 et un composé de formule III
R-OH III
dans les formules I à III, le radical R représentant H ou un radical alkyle contenant 1 ou 2 atomes de carbone, le radical R1 représentant H, un radical méthyle ou éthyle, et les radicaux R2 et R3 représentant des radicaux méthyle ou éthyle, les radicaux R2 et R3 pouvant être identiques ou différents,
**caractérisé en ce qu'**une composition d'oxyde mixte selon les revendications précédentes est utilisée en tant que catalyseur.

6. Procédé selon la revendication 5, **caractérisé en ce qu'**une composition d'oxyde mixte fabriquée par hydrolyse à la flamme est utilisée en tant que catalyseur, qui a la composition suivante :
a. silicium : 50 à 99,9 % en masse (calculé en tant que SiO₂),
b. aluminium : 0,1 à 50 % en masse, de préférence 0,1 à 20 % en masse, de manière particulièrement préférée 1 à 11 % en masse (calculé en tant qu'Al₂O₃),
c. métal alcalin : 0 à 15 % en masse (calculé en tant que M₂O) ou métal alcalino-terreux : 0 à 30 % en masse (calculé en tant que MO).

7. Procédé selon la revendication 5 ou 6, dans lequel de l'éther de méthyle et de tert.-butyle est utilisé en tant que matériau de départ.

8. Procédé selon la revendication 5 ou 6, dans lequel de l'alcool tert.-butylique est utilisé en tant que matériau de départ.

9. Procédé selon la revendication 8, dans lequel du tert.-butanol en mélange azéotropique avec de l'eau est utilisé en tant que matériau de départ.

10. Procédé selon la revendication 5 ou 6, dans lequel la température se situe dans une plage allant de 110 à 450 °C.

11. Procédé selon la revendication 5 ou 6, dans lequel la pression se situe dans une plage allant de 0,1 à 2 MPa (abs).
